Europäisches Patentamt

(19) European Patent Office

Office européen des brevets

(11) Numéro de publication: **0 056 563**
**B1**

(12) **FASCICULE DE BREVET EUROPEEN**

(45) Date de publication du fascicule du brevet:
04.09.85

(51) Int. Cl.⁴: **A 61 B 5/14**

(21) Numéro de dépôt: 81810499.4

(22) Date de dépôt: 16.12.81

(54) **Dispositif pour déterminer le groupe sanguin d'un individu.**

(30) Priorité: 19.01.81 CH 306/81

(43) Date de publication de la demande:
28.07.82 Bulletin 82/30

(45) Mention de la délivrance du brevet:
04.09.85 Bulletin 85/36

(84) Etats contractants désignés:
AT BE CH DE FR GB IT LI LU NL SE

(56) Documents cités:
FR - A - 1 407 345
FR - A - 2 307 258
FR - A - 2 361 651
US - A - 3 395 629
US - A - 3 453 941
US - A - 3 532 470
US - A - 3 698 822
US - A - 3 876 377
US - A - 4 079 393
US - A - 4 135 818

(73) Titulaire: **Société FINAMEX Finance Corporation, Calle Aquilino de la Guardia, 8 Apartado 850, Ciudad de Panama (PA)**

(72) Inventeur: **Zanchi, Jean, Route de Sébeilion 7, CH-1004 Lausanne (CH)**

(74) Mandataire: **Nithardt, Roland, CABINET ROLAND NITHARDT Rue Edouard Verdan 15, CH-1400 Yverdon (CH)**

## Description

La présente invention concerne un dispositif pour déterminer le groupe sanguin d'un individu, comportant une installation pour prélever et analyser automatiquement des échantillons de sang de cet individu, cette installation comprenant des organes agencés pour recevoir des récipients d'analyse associés à des doses déterminées de réactifs, pour prélever des quantités déterminées de sang de l'individu et les déposer dans ces récipients, pour mélanger automatiquement les doses de réactifs auxdites quantités déterminées du sang pour analyser les réactions obtenues, un dispositif pour fournir les résultats des analyses et un dispositif photographique pour prendre une photographie de l'individu concerné, comportant des organes agencés pour associer à la photographie de l'individu une représentation des résultats des analyses.

La détermination des antigènes portés par les hématies du sang est obtenue en mélangeant à ce sang des anticorps connus, dénommés sérum anti A, sérum anti B, sérum anti AB et sérum anti D, qui provoquent l'agglutination des hématies par l'anticorps correspondant.

On connaît déjà des appareils automatiques permettant de réaliser ces analyses et d'adjoindre aux résultats la photographie de l'individu, dont l'opérateur a analysé le sang.

Ces dispositifs sont destinés à permettre d'éviter les erreurs de repérage d'un groupe sanguin et de supprimer les conséquences très graves qu'elles peuvent provoquer en cas de transfusion sanguine. Bien que l'idée d'adjoindre la photographie d'un individu à une plaquette montrant le résultat de son analyse sanguine constitue un progrès évident tendant à diminuer ces risques d'erreur, cette solution n'élimine toutefois pas toutes les erreurs provenant soit de la manipulation des sérums, soit de celle des plaques d'analyse, soit de la distribution erronée d'une plaquette d'analyse à un individu.

Le brevet US-A-3 453 941 décrit par exemple un appareil du type mentionné ci-dessus, dans lequel un groupe mobile de pipettes, relié à une pompe volumétrique stationnaire, prélève les sérums dans des flacons, puis du sang sur un doigt du patient et dépose ces mélanges dans des cavités d'une plaquette d'analyse où s'effectue le test d'agglutination. Le résultat de ce test, sous la forme d'une image de la plaquette d'analyse et d'une plaquette portant la désignation du groupe sanguin déterminé par l'opérateur, est transmis par un dispositif optique à un appareil photographique qui enregistre, sur le même film que cette image, une photographie de l'individu.

Cependant, un tel appareil ne garantit pas une détermination suffisamment sûre et objective du groupe sanguin, principalement parce qu'il ne fournit pas des photographies de la plaquette qui soient suffisamment précises pour permettre de confirmer la détermination du groupe sanguin faite par l'opérateur. En outre, l'appareillage proposé n'est pas en mesure d'empêcher des erreurs de manipulation des sérums ni d'assurer un dosage très précis du mélange. Enfin, comme les pipettes véhiculent les mélanges de sang et de sérums, l'agglutination commence déjà dans les pipettes, de sorte que des résidus agglutinés peuvent s'y former et n'être pas transférés sur la plaquette d'analyse.

Le brevet français publié sous le No. 2 296 869 décrit aussi un appareil tendant à automatiser l'adjonction de la photographie d'un individu aux résultats d'une analyse pour la détermination du groupe sanguin. A cet effet, l'installation correspondante comporte un appareil photographique pour la prise d'une photographie de l'individu et la photographie de la plaquette d'agglutination correspondante. Cette plaquette est transparente, et un dispositif optique projette par transparence l'image des réactions sanguines de la plaquette sur un écran, de telle manière que l'appareil photographique de l'installation puisse photographier simultanément l'écran et l'individu.

Un risque subsiste cependant pour ce système: celui de l'interprétation fausse de la photographie des résultats d'analyse, notamment lorsque la reproduction photographique n'est pas parfaitement nette. Par ailleurs, l'appareil permettant d'effectuer les analyses susmentionnées est d'une conception extrêmement compliquée et ne remplit plus de façon absolue les conditions d'hygiène moderne actuellement imposées pour de telles analyses.

La présente invention se propose de pallier ces différents inconvénients en réalisant notamment un dispositif tel que susmentionné, caractérisé en ce que le dispositif pour fournir le résultat des analyses comporte un dispositif de mesure de la densité optique du contenu des récipients d'analyse et un appareil enregistreur agencé pour transcrire les résultats de cette mesure sous forme d'un graphique constituant ladite représentation des résultats des analyses.

La présente invention, ainsi que ses principaux avantages, seront décrits plus en détail en référence à la description d'un exemple de réalisation préféré et du dessin annexé dans lequel:

La figure 1 représente une vue schématique en perspective d'une forme de réalisation préférée de l'appareil selon l'invention,

Les figures 2, 2A et 2B représentent différents aspects d'une plaquette d'analyse utilisée dans l'appareil selon l'invention,

Les figures 3 et 3A représentent deux vues d'une cassette contenant une bande de plaquettes d'analyse selon la fig. 2,

La figure 4 représente une vue détaillée agrandie des capillaires de prises de sang utilisés dans l'appareil de la fig. 1,

La figure 5 représente un dispositif de lecture optique du niveau du sang dans les capillaires de la fig. 1,

La figure 6 représente une vue schématique du dispositif de déposition du sang dans les cuvettes d'analyses,

La figure 7 représente un schéma de fonctionnement du dispositif de la fig. 6,

La figure 8 représente une phase de l'opération de déposition du sang dans les cuvettes,

La figure 9 illustre deux phases successives de l'adjonction des réactifs au sang contenu dans les cuvettes,

La figure 10 représente un schéma du dispositif de mesure de la densité optique du mélange sangantisérum,

La figure 11 représente une vue agrandie des érythocytes agglutinés, disposés dans la cuvette,

La figure 12 représente le schéma de principe de la mesure telle que réalisée par le dispositif de la fig. 10, dans le cas d'une agglutination et celui d'une non-agglutination,

La figure 13 représente le résultat de l'intégration des mesures optenues conformément au principe illustré par la fig. 12,

La figurine 14 illustre une carte combinant la photographie de l'individu dont l'analyse de sang a été effectuée, sa fiche signalétique et une représentation graphique des résultats de l'analyse du sang, et

Les figures 15 à 18 illustrent différents systèmes périphériques pour lesquels le dispositif selon l'invention est prévu.

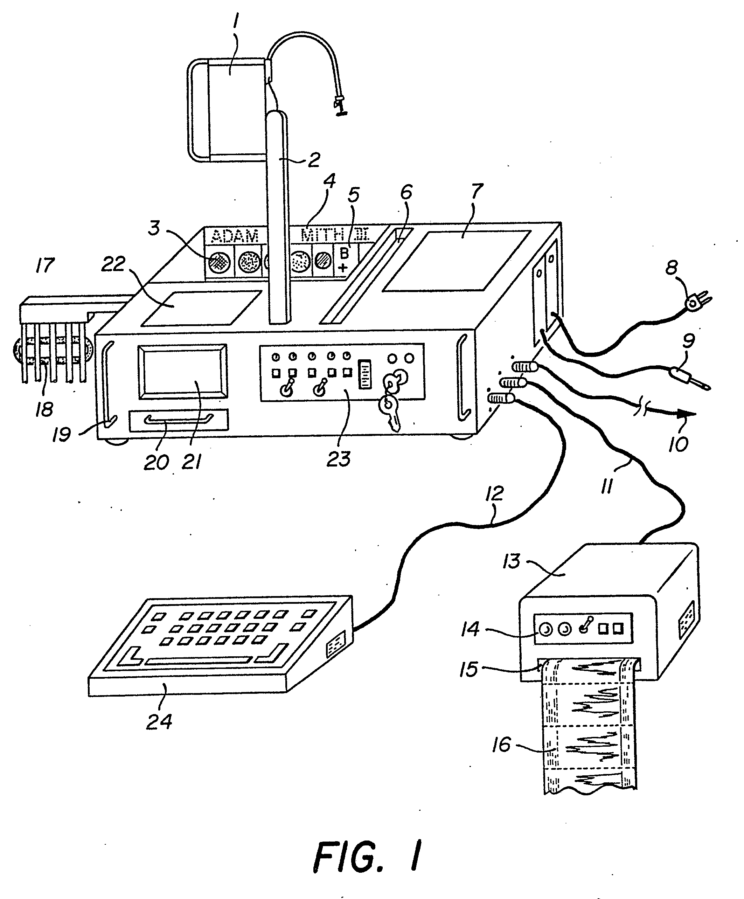

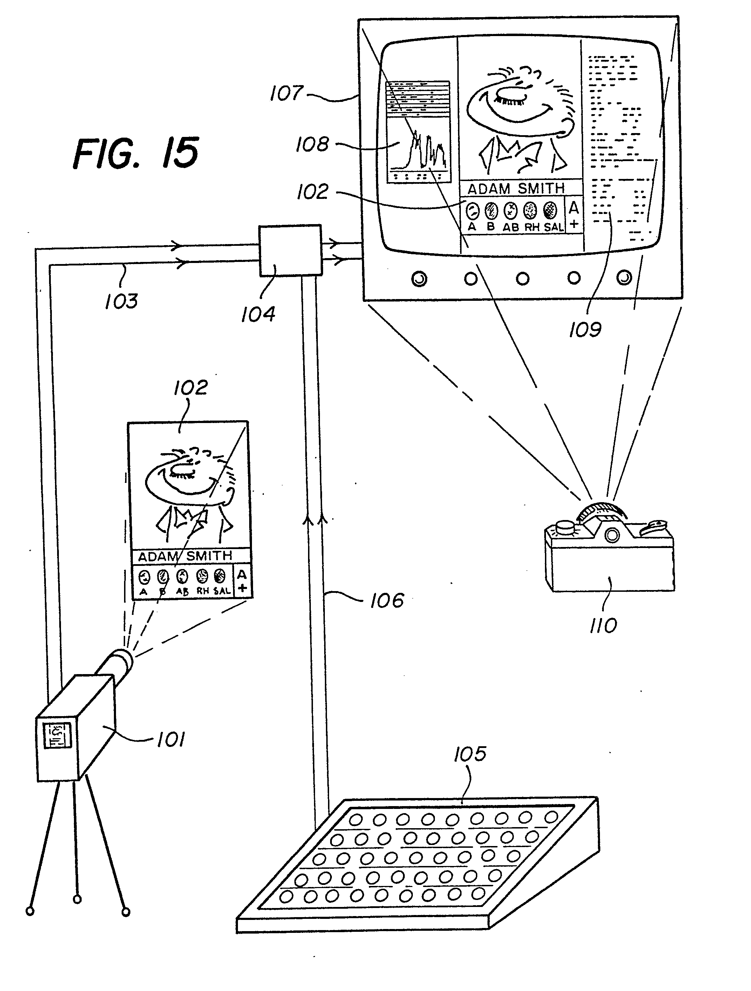

En référence à la fig. 1, le dispositif pour déterminer le groupe sanguin d'un individu, tel que défini précédemment, comporte essentiellement un groupe photographique 1 monté sur un pied du type pied universel 2, solidaire du boîtier du dispositif. Un écran 3, solidaire d'une des parois arrières du boîtier, est prévu pour recevoir en projection les résultats des tests, projetés en transparence sur cet écran. Cet écran est surmonté d'un dispositif d'affichage 4 à cristaux liquides, qui permet de visualiser le nom et le prénom de l'individu concerné. Un second dispositif d'affichage 5, pouvant également être un dispositif d'affichage à cristaux liquides, est prévu pour afficher le groupe sanguin et le rhésus. Le groupe photographique 1 permet de photographier simultanément l'écran 3 et un individu placé derrière et audessus de cet écran pour établir une carte de groupe sanguin telle que repŕsentée par la fig. 15. Ce groupe est de préférence conçu pour photographier également simultanément les graphiques de la bande 16, l'ensemble des données photographiées simultanément constituant la fiche signalétique médicale de l'individu.

Dans le boîtier du dispositif, est ménagée une cavité prévue pour permettre le logement du pied universel 2 pendant le transport, et une niche 7 est prévue pour recevoir le groupe photographique lorsque cet ensemble est démonté.

Deux prises 8 et 9 permettent l'alimentation du dispositif respectivement en courant alternatif ou en courant continu. Des niches ménagées dans le boîtier permettent de loger les câbles correspondants. Sur la même paroi du boîtier, sont également prévues une sortie 10 pour le raccordement à un appareil périphérique, une sortie 11 pour le raccordement à l'unité imprimante 13 et une sortie 12 pour le raccordement avec un clavier de commande 24.

L'unité imprimante 13 comporte des touches de commande 14 rassemblées sur un tableau, et permet de fournir une représentation graphique des tests effectués, enregistrés sur une bande 16 d'un support quelconque, notamment en papier perforé.

Un bras porteur 17 de capillaires 18 permet d'effectuer les prises de sang, comme cela sera décrit plus en détail par la suite. La face avant du boîtier comporte des poignées de transport 19, un tiroir 20 pour entreposer les tests utilisés, un écran transparent 21 pour permettre à l'opérateur de suivre les déroulements des analyses, et un tableau 23 sur lequel sont montés les organes de commande. Un couvercle 22 permet d'accéder au dispositif de changement des cassettes (décrit par la suite) contenant les plaquettes d'analyse.

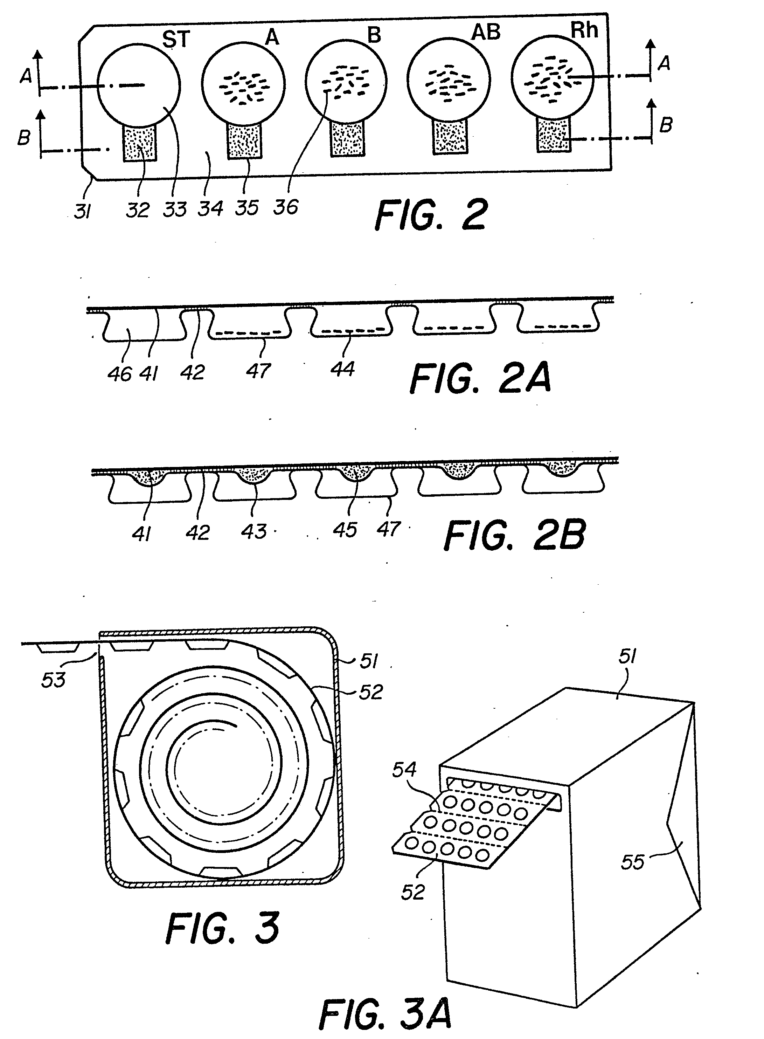

La fig. 2 représente une vue de dessus d'une forme de réalisation préférée des plaquettes d'analyse utilisées par le dispositif décrit ci-dessus. Ces plaquettes d'analyse se présentent sous la forme d'un rectangle biseauté en 31, pour éviter une introduction erronée sur la table d'analyse. Une solution physiologique 32, à 9% de chlorure de sodium (NaCl) est contenue dans des pochettes 35 en matière plastique souple. Les cuvettes 33, servant à l'analyse, sont formées au moyen d'une matière synthétique thermoformable. La première cuvette, destinée à contenir le test de référence, ne contient aucun antisérum. Par contre, les autres cuvettes contiennent toutes un antisérum 36, de préférence sous forme lyophilisée. Une couche en aluminium 34 recouvre et scelle les cuvettes 33 et les pochettes 35.

Les fig. 2A et 2B représentent respectivement des vues en coupe selon les lignes A–A et B–B de la plaquette de la fig. 2. Une couche de colle synthétique 42 permet de relier la couche d'aluminium 41 aux surfaces en regard des cuvettes 43. Les antisérums lyophilisés 44 sont disposés au fond des cuvettes 47, et la solution physiologique 45 est placée dans les pochettes 43. La cuvette 46 ne contient pas d'antisérum et sert comme référence pour les tests.

Comme le montrent plus précisément les fig. 3 et 3A, les plaquettes individuelles se présentent de préférence sous la forme de bandes enroulées 52, logées dans une cassette 51 comportant une ouverture de sortie 53 de la bande. Les plaquettes individuelles sont détachées selon la perforation 54. La cassette 51 a un profil et comporte notamment un évidement 55, qui empêche l'introduction erronée de la cassette dans le dispositif. Les cassettes sont préemballées. Les cuvettes contiennent l'antisérum sous forme lyophilisée, ce qui lui donne une durée de vie quasi illimitée. Les fluctuations de température et d'humidité de l'environnement ne l'influencent guère. A l'intérieur des cassettes, ils sont protégés contre la lumière, notamment contre les rayons UV. Comme cela sera décrit plus en détail par la suite, au moment de l'analyse, les pochettes, contenant la solution physiologique, sont brisées. Avec l'antisérum lyophilisé, la solution physiologique composera un antisérum liquide prêt à agir sur les échantillons de sang prélevés.

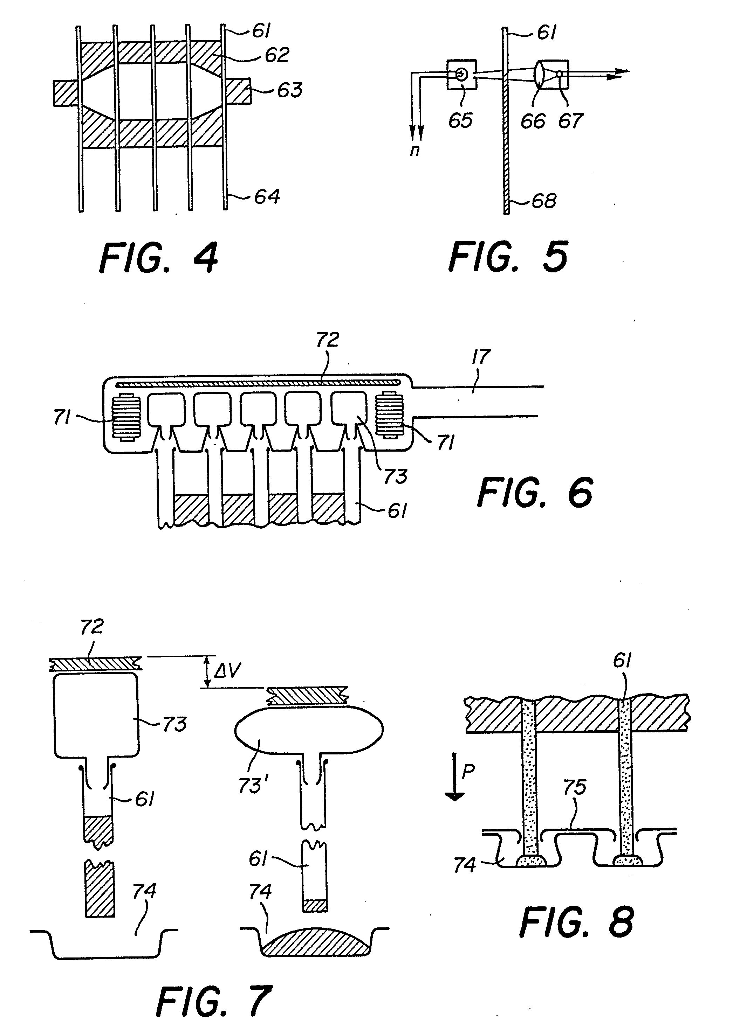

La fig. 4 représente le dispositif de prises de sang. A cet effet, on utilise un jeu de cinq capillaires 61 liés entre eux par des ponts 62, et comportant de part et d'autre deux prises 63 pour faciliter la manipulation. L'extrémité inférieure 64 du jeu de capillaires 61 est destinée à être introduite dans le bras porteur, et à en être retirée d'une façon parfaitement hygiénique.

La fig. 5 illustre un dispositif de lecteur optique permettant de vérifier la quantité de sang introduite dans chacun des capillaires. Un capillaire 61 est placé dans le champ d'une source de lumière 65, dont le faisceau est concentré par une lentille 66 sur une cellule photo-électrique 67. Cette cellule fournit un signal lorsque le niveau du sang 68, dans le capillaire 61, coupe le faisceau émis par la source 65. Le dispositif peut être conçu de manière à émettre un signal sonore, qui indique à l'opérateur que le niveau est atteint et lui permet de passer au capillaire suivant.

Pour déposer le sang dans les cuvettes correspondantes, le bras 17, portant les capillaires 61, contient deux électro-aimants 71, qui coopèrent avec une plaquette métallique 72 (fig. 6) qui agit en compression sur des bulbes souples 73, communiquant avec l'extrémité supérieure des capillaires 61.

La fig. 7 représente plus en détail l'aspect des bulbes au cours de l'opération de déposition du sang. La plaquette métallique 72, actionnée par les électro-aimants, exerce une poussée sur les bulbes 73, qui s'écrasent pour adopter une forme aplatie telle que représentée en 73'. Cet écrasement correspond à un déplacement en hauteur Δv de la plaquette métallique 72, qui a pour effet de refouler le sang contenu dans les capillaires 61, pour le déposer dans les cuvettes 74. Ce dépôt s'effectue après que le jeu de capillaires soit descendu selon la flèche P (fig. 8) pour percer la couche d'aluminium 75 qui scelle à l'origine les cuvettes 74.

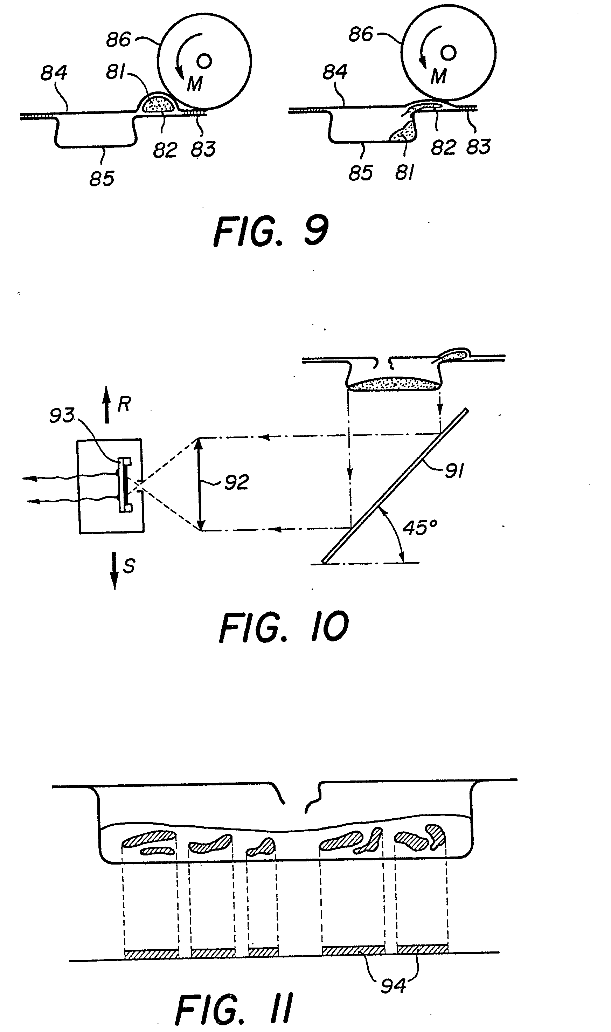

La fig. 9 illustre plus en détail l'opération consistant à ajouter la solution physiologique à l'antisérum lyophilisé contenu dans la cuvette. La solution physiologique 81 est contenue dans une pochette 82 et recouverte par la feuille d'aluminium collée à la matière plastique de base par une couche de colle 83. La feuille d'aluminium 84 recouvre la cuvette 85. Un rouleau 86, se déplaçant dans le sens de la flèche M, écrase la pochette 82 et déverse la solution physiologique 81 dans la cuvette 85. L'échantillon de sang, l'antisérum lyophilisé et la solution physiologique sont mélangés à l'intérieur de la cuvette 85 sous l'effet d'ultrasons, ce qui permet un mixage intense, sans toutefois briser les cellules ou les macromolécules.

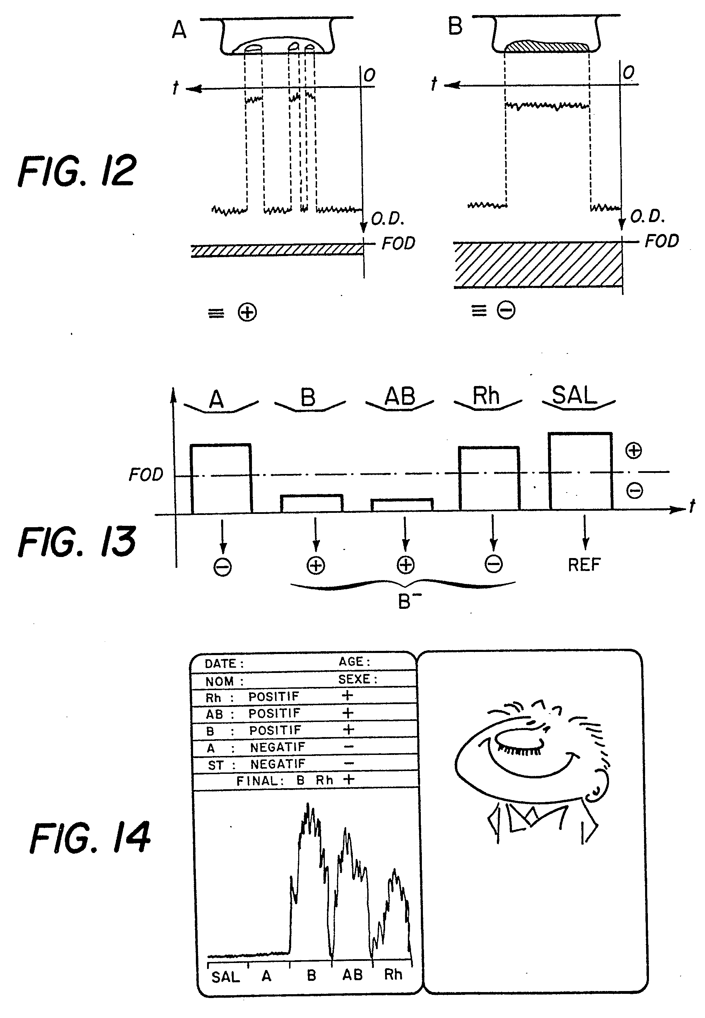

Le dispositif décrit évalue les résultats du test d'agglutination, en mesurant la densité optique du mélange sang/antisérum. A cet effet, le dispositif de mesure, décrit en référence à la fig. 10, comporte un miroir 91 disposé à quarante-cinq degrés, qui renvoie l'image du contenu de la cuvette (représentée fortement agrandie par la fig. 11) sur une optique 92, focalisant le faisceau sur une cellule SBC (Silicone Blue Cell) 93. Cette cellule se déplace dans le sens des flèches R et S, pour effectuer un balayage permettant d'obtenir des signaux proportionnels aux variations de densité du mélange sang/antisérum. La fig. 11 montre en 94 les projections verticales des zones à forte densité dans la cuvette examinée.

Les fig. 12A et 12B représentent graphiquement la densité optique (O.D.) en fonction du temps t. Les lignes FOD représentent l'intégration effectuée par un circuit électronique correspondant de la série des densités mesurées. La valeur de cette intégration indique qu'il s'agit d'une agglutination (réaction positive fig. 12A) ou d'une non-agglutination (réaction négative fig. 12B). Comme valeur de référence, on prend la densité mesurée dans la cuvette de référence ne contenant pas d'antisérum. Ce dispositif permet de détecter de façon absolue même la plus faible des agglutinations (par exemple en rhésus), comme illustré à grande échelle par la fig. 13.

Le dispositif enregistreur mentionné précédemment permet d'obtenir l'enregistrement sur papier du graphique des résultats des analyses. Ces graphiques s'inscrivent sur une bande de papier portant toutes les informations utiles correspondant à l'individu dont l'opérateur a effectué l'analyse sanguine; par exemple, son nom, son prénom, son âge, son sexe, etc., ce qui constitue sa fiche signalétique civile, ainsi que toutes les données résultant des analyses susmentionnées. Le groupe photographique du dispositif décrit est agencé de telle manière qu'on puisse photographier simultanément la fiche signalétique civile et la fiche signalétique médicale, de façon à émettre une carte telle que représentée par la fig. 14. Cette carte peut se présenter sous différentes formes, par exemple elle peut comporter deux volets, la photographie de l'individu étant disposée à côté de sa fiche signalétique, ou un seul volet, la photographie se trouvant au verso de la carte portant la fiche signalétique et les résultats graphiques des tests. Les informations relatives à l'individu sont remplies à l'aide du clavier 24 représenté par la fig. 1.

Les fig. 15, 16, 17 et 18 représentent différents systèmes périphériques, dans lesquels le dispositif décrit peut être incorporé. Plus particulièrement, en référence à la fig. 15, ce dispositif comporte une caméra de télévision 101 destinée à filmer la photographie et les résultats d'analyse 102 d'un individu. L'image obtenue sera transmise par un câble 103 à une table mélangeuse 104. Un clavier 105 permet d'introduire d'autres données par le câble 106 dans la table mélangeuse 104, qui est directement reliée à un poste de télévision 107. L'ensemble des informations, apparaissant sur l'écran et comportant d'une part l'image de la fiche 102, d'autre part la reproduction 108 des résultats graphiques des analyses et d'autres informations 109 touchant l'individu, sont photographiées par une caméra 110 qui peut être couplée à différents dispositifs connus en soi.

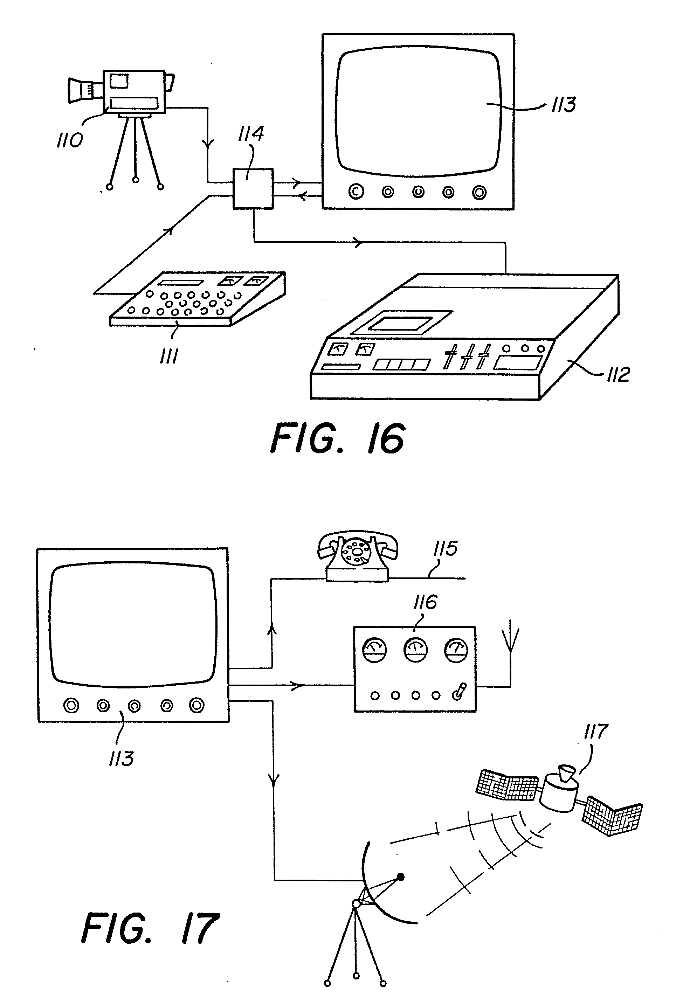

En référence à la fig. 16, la caméra 110 peut être placée à l'entrée d'un dispositif de mémorisation comportant un clavier de commande 111, un enregistreur à cassettes 112 et un poste de télévision 113, ces éléments étant reliés entre eux par un circuit électronique 114. En référence à la fig. 17, les informations mémorisées et visualisées sur le poste de télévision 113 peuvent être transmises par toutes les voies de télécommunication couramment utilisées, soit le téléphone 115, le télex 116 ou les transmissions par relais satellite 117.

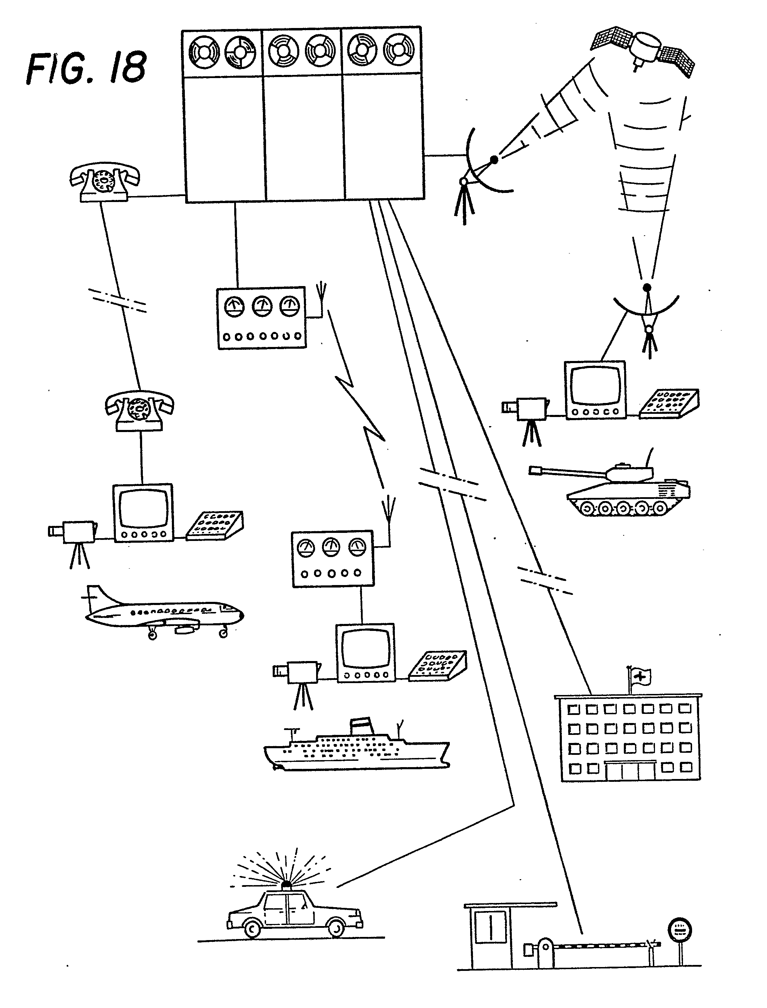

La fig. 18 illustre une généralisation du dispositif grâce auquel il serait possible de rassembler, dans un ordinateur central, les fiches signalétiques des individus et d'installer des terminaux, par exemple dans des postes de secours ou des hôpitaux.

**Revendications**

1. Dispositif pour déterminer le groupe sanguin d'un individu, comportant une installation pour prélever et analyser automatiquement des échantillons de sang de cet individu, cette installation comprenant des organes agencés pour recevoir des récipients d'analyse (33) associés à des doses déterminées de réactifs, pour prélever des quantités déterminées de sang de l'individu et les déposer dans ces récipients, pour mélanger automatiquement les doses de réactifs auxdites quantités déterminées du sang et pour analyser les réactions obtenues, un dispositif (3, 13) pour fournir les résultats des analyses et un dispositif photographique (1) pour prendre une photographie de l'individu concerné, comportant des organes agencés pour associer à la photographie de l'individu une représentation des résultats des analyses, caractérisé en ce que le dispositif pour fournir les résultats des analyses comporte un dispositif de mesure (92, 93) de la densité optique du contenu des récipients d'analyse (33) et un appareil enregistreur (13) agencé pour transcrire les résultats de cette mesure sous forme d'un graphique constituant ladite représentation des résultats des analyses.

2. Dispositif selon la revendication 1, caractérisé en ce que les graphiques des résultats de mesure de densité optique sont enregistrés sur un support, et en ce que le dispositif photographique permet de photographier simultanément au moins ce support et l'individu.

3. Dispositif selon la revendication 1, caractérisé en ce que les récipients d'analyse sont constitués par des cuvettes (33) en matière synthétique, embouties par thermoformage et disposées par rangées sur une bande enroulée (52) contenue dans une cassette (51).

4. Dispositif selon la revendication 3, caractérisé en ce que les cuvettes (33) sont recouvertes d'une feuille d'aluminium (34).

5. Dispositif selon la revendication 3, caractérisé en ce que chaque rangée comporte une cuvette de référence (46) et au moins une cuvette d'analyse (47) contenant un antisérum (44) à l'état lyophilisé.

6. Dispositif selon la revendication 3, caractérisé en ce qu'à chaque cuvette (33) est associée une pochette souple (35) contenant une solution physiologique (32), cette pochette étant prévue pour être écrasée de telle manière que le liquide physiologique s'écoule dans la cuvette et constitue, avec l'antisérum lyophilisé, un antisérum liquide.

7. Dispositif selon la revendication 1, caractérisé en ce que les organes pour prélever automatiquement les échantillons de sang comportent une série de capillaires (61) disposés parallèlement et noyés dans un support, et un dispositif optique (65 et 67) permettant de détecter le niveau du sang dans les capillaires.

8. Dispositif selon la revendication 1, caractérisé en ce que les organes, destinés à déposer automatiquement les échantillons de sang dans les cuvettes, comportent des bulbes souples (73) associés à chaque capillaire (61), et un dispositif électromécanique (71, 72) pour écraser au moins partiellement les bulbes souples, pour provoquer l'écoulement du sang contenu dans les capillaires.

9. Dispositif selon la revendication 1, caractérisé en ce que les organes prévus pour mélanger les échantillons de sang, le liquide physiologique et l'antisérum lyophilisé contenu dans les cuvettes comportent un générateur à ultrasons.

10. Dispositif selon la revendication 1, caractérisé en ce qu'il comporte des organes périphériques (101, 107, 110, 112) permettant de produire, emmagasiner et transmettre le groupe d'informations comprenant au moins la photographie de l'individu, les résultats de l'analyse pour la dédermination du groupe sanguin et les graphiques illustrant les résultats de cette analyse.

**Claims**

1. Apparatus for determining the blood type of an individual, comprising a device for taking and automatically analysing said individual's blood samples, this device comprising means disposed to receive receptacles for analysis (33) combined with predetermined doses of reagents, to take predetermined quantities of the individual's blood and to deposite its in the receptacles, to automatically mix the doses of the reagents with said predetermined quantities of blood and to analyse the reactions obtained, an apparatus (3, 13) for furnishing the results of the analysis and a photographic apparatus (1) to take a photograph of the individual concerned, comprising means to combine a photograph of the individual with a graph of the results of the analysis, characterized in that the apparatus for furnishing the results of the analysis comprises a device (92, 93) for measuring the optical density of the contents of the analysis receptacles (33) and a recording device (13) to transcribe said results of density measurement in the form of graph constituting said representation of the results of analysis.

2. Apparatus according to claim 1, characterized in that graphs of the results of optical density measurements are recorded on a support and in that the photographic apparatus allows the simultaneous photographing of said support holding the graphs, of the individual and of his identification card.

3. Apparatus according to claim 1, characterized in that the receptacles for analysis (33) are bulb-like, of heat-stamped synthetic material and disposed in rows on a rolled up strip (52) contained in a cassette (51).

4. Apparatus according to claim 3, characterized in that the receptacles (33) are covered with a aluminium foil layer (34).

5. Apparatus according to claim 3, characterized in that each row comprises a reference receptacle (46) and at least one analysis receptacle (47) containing an anti-serum (44) in the lyophilized state.

6. Apparatus according to claim 3, characterized in that each receptacle (33) is associated with a pliable pouch (35) containing a physiological solution (32), said pouch being provided so it can be crushed in such a manner that the physiological solution flows into the receptacle and forms, with a lyophilized anti-serum, a liquid anti-serum.

7. Apparatus according to claim 1, characterized in that the means for automatically taking the blood samples comprises a series of tubes (61) in parallel disposition and held in a support, and an optic means (65 to 67) allowing detection of the level of blood in said tubes.

8. Apparatus according to claim 1, characterized in that the means for automatically depositing the blood samples in the receptacles comprises pliable bulbs (73) associated with each tube (61), and an electromechanical means (71, 72) to at least partially flatten the pliable bulbs to cause the blood contained in said tubes to flow out.

9. Apparatus according to claim 1, characterized in that the means provided to mix the blood samples, the physiological liquid and the lyophilized anti-serum contained in the receptacles comprises an ultrasound generator.

10. Apparatus according to claim 1, characterized in that it comprises peripheral devices (101, 107, 110, 112) allowing reproduction, storage and transmission of the group of information comprising at least the individual's photograph, his identification card and the graphs illustrating the test results.

**Patentansprüche**

1. Apparat zur Blutgruppenbestimmung einer Person, der eine Einrichtung zur automatischen Entnahme und Analyse der Blutmuster dieser Person aufweist, wobei diese Einrichtung Mittel umfasst, die zur Aufnahme von Analysegefässen (33) vorgesehen sind, die bestimmten Reagenzdosen zugeordnet sind, zur Entnahme bestimmter Blutmengen von der Person und zum Absetzen derselben in diesen Gefässen, zum automatischen Beimischen der Reagenzdosen den genannten bestimmten Blutmengen und zur Analyse der erhaltenen Reaktionen, sowie eine Vorrichtung (3, 13) zum Liefern der Analyseergebnisse und eine photographische Einrichtung (1) zum Aufnehmen einer Photographie der betreffenden Person, wobei sie Mittel umfasst, die vorgesehen sind um der Photographie der Person eine Darstellung der Analyseergebnisse zuzuordnen, dadurch gekennzeichnet, dass die Vorrichtung zum Liefern der Analyseergebnisse eine Messvorrichtung (92, 93) zum Messen der optischen Dichte des Inhaltes der Analysegefässe (33) sowie ein Registriergerät (13) umfasst, das zur Übertragung der Ergebnisse dieser Messung in Form einer graphischen Darstellung der Analyseergebnisse vorgesehen ist.

2. Apparat nach Anspruch 1, dadurch gekennzeichnet, dass die graphische Darstellung der Ergebnisse der optischen Dichtemessung auf einem Träger registriert sind, und dass die photographische Einrichtung es erlaubt gleichzeitig mindestens diesen Träger und die Person zu photographieren.

3. Apparat nach Anspruch 1, dadurch gekennzeichnet, dass die Analysegefässe aus Kunststoffküvetten bestehen, die durch thermische Verformung gepresst und reihenweise auf einem aufgerollten Band (52) in einer Kassette (51) angeordnet sind.

4. Apparat nach Anspruch 3, dadurch gekennzeichnet, dass die Küvetten (33) mit einer Aluminiumfolie (34) bedeckt sind.

5. Apparat nach Anspruch 1, dadurch gekennzeichnet, dass jede Reihe eine Bezugsküvette (46) und mindestens eine Analyseküvette (47) umfasst, die ein Antiserum (44) im lyophilisierten Zustand enthält.

6. Apparat nach Anspruch 3, dadurch gekennzeichnet, dass jeder Küvette (33) eine biegsame Tasche (35) zugeordnet ist, die eine physiologische Lösung (32) enthält, wobei diese Tasche vorgesehen ist um zerdrückt zu werden, sodass die physiologische Flüssigkeit in die Küvette abfliesst und mit dem lyophilisierten Antiserum ein flüssiges Antiserum bildet.

7. Apparat nach Anspruch 1, dadurch gekennzeichnet, dass die Mittel zur automatischen Blutmusterentnahme eine Reihe von Kapillaren (61) aufweisen, die parallel in einem Träger versenkt angeordnet sind, und eine optische Einrichtung (65 bis 67), die den Blutspiegel in den Kapillaren anzuzeigen erlaubt.

8. Apparat nach Anspruch 1, dadurch gekennzeichnet, dass die zum automatischen absetzen der Blutmuster in den Küvetten bestimmten Mittel biegsame Wülste (73) aufweisen, die jeder Kapillaren (61) zugeordnet sind, und eine elektromechanische Vorrichtung (71, 72) um die biegsamen Wülste mindestens teilweise zu zerdrücken und das Abfliessen des in den Kapillaren enthaltenen Blutes zu bewirken.

9. Apparat nach Anspruch 1, dadurch gekennzeichnet, dass die zum Mischen der Blutmuster, der physiologischen Flüssigkeit und des in den Küvetten enthaltenen lyophilisierten Antiserums

vorgesehenen Mittel einen Ultraschallgenerator umfassen.

10. Apparat nach Anspruch 1, dadurch gekennzeichnet, dass er Zusatzmittel (101, 107, 110, 112) umfasst, die die Erzeugung, Speicherung und Übertragung der Informationsgruppe erlauben, die mindestens eine Photographie der Person, die Analyseergebnisse zur Blutgruppenbestimmung und die graphische Darstellung der Ergebnisse dieser Analyse umfasst.

## FIG. 1

FIG. 2

FIG. 2A

FIG. 2B

FIG. 3

FIG. 3A

FIG. 4

FIG. 5

FIG. 6

FIG. 7

FIG. 8

FIG. 9

FIG. IO

FIG. II

## FIG. 12

A

B

$\equiv \oplus$

$\equiv \ominus$

## FIG. 13

A    B    AB    Rh    SAL

FOD

$\oplus$

$\ominus$

$\ominus$    $\oplus$    $\oplus$    $\ominus$    REF

$B^-$

## FIG. 14

| DATE : | AGE : | |
|---|---|---|
| NOM : | SEXE : | |
| Rh : | POSITIF | + |
| AB : | POSITIF | + |
| B : | POSITIF | + |
| A : | NEGATIF | − |
| ST : | NEGATIF | − |
| FINAL: B Rh + | | |

SAL    A    B    AB    Rh

FIG. 15

107
108
102
109
103
104
102
106
105
101
110

FIG. 16

FIG. 17

FIG. 18

0 056 563